(19) Europäisches Patentamt
European Patent Office
Office européen des brevets

(11) Veröffentlichungsnummer: **0 007 568**
**A2**

(12) # EUROPÄISCHE PATENTANMELDUNG

(21) Anmeldenummer: **79102519.0**

(22) Anmeldetag: **18.07.79**

(51) Int. Cl.³: A 01 N 41/08
C 07 C 161/00

(30) Priorität: **02.08.78 DE 2833856**

(43) Veröffentlichungstag der Anmeldung:
**06.02.80 Patentblatt 80/3**

(84) Benannte Vertragsstaaten:
**BE CH DE FR GB IT NL**

(71) Anmelder: **BAYER Aktiengesellschaft**
**Zentralbereich Patente,Marken und Lizenzen Bayerwerk**
**D-5090 Leverkusen 1(DE)**

(72) Erfinder: **Hofer, Wolfgang, Dr.**
**Pahlkestrasse 59**
**D-5600 Wuppertal 1(DE)**

(72) Erfinder: **Lürssen, Klaus, Dr.**
**August-Kierspel-Strasse 89**
**D-5060 Bergisch-Gladbach(DE)**

(54) **Halogenäthyl-thiosulfonsäureester enthaltende Mittel zur Regulierung des Pflanzenwachstums und ihre Verwendung.**

(57) Die teilweise bekannten Halogenäthyl-thiosulfonsäureester der Formel

$$\text{Hal-CH}_2\text{CH}_2\text{-S(O)}_m\text{-S(O)}_n\text{-R} \quad (I)$$

in welcher
R für Alkyl, Halogenalkyl oder gegebenenfalls substituiertes Aryl steht,
Hal für Chlor oder Brom steht und
m und n immer verschieden sind und für

Null oder 2 stehen,

eignen sich sehr gut zur Regulierung des Pflanzenwachstums.

EP 0 007 568 A2

Croydon Printing Company Ltd.

BAYER AKTIENGESELLSCHAFT
Zentralbereich
Patente, Marken und Lizenzen

5090 Leverkusen, Bayerwerk
Dü/Drey
II b

BEZEICHNUNG GEÄNDERT
siehe Titelseite

Mittel zur Regulierung des Pflanzenwachstums

Die vorliegende Erfindung betrifft die Verwendung teilweise bekannter Halogenäthyl-thiosulfonsäureester als Wirkstoffe zur Regulierung des Pflanzenwachstums.

Es ist bereits bekannt geworden, daß bestimmte Verbindungen, die eine 2-Chloräthylgruppe enthalten, pflanzen wachstumsregulierende Eigenschaften besitzen. So können (2-Chloräthyl)-trimethylammonium-chlorid und 2-Chloräthylphosphon-säure zur Regulierung des Pflanzenwachstums verwendet werden (vgl.US-PS 3 156 554 und Nature 218 (1968), 974). Die Wirksamkeit dieser Stoffe ist jedoch - vor allem bei niedrigen Aufwandmengen- nicht immer ganz befriedigend.

Bekannt ist auch die fungizide und bakterizide Wirkung von bestimmten Chloralkyl-thiosulfonsäureestern, wie zum Beispiel von Methyl-thiosulfonsäure-S-(2-chloräthyl-ester (vgl. US-PS 3 275 506).

Es wurde nun gefunden, daß die teilweise bekannten Halogenäthyl-thiosulfonsäureester der Formel

$$Hal-CH_2CH_2-S(O)_m-S(O)_n-R \quad (I)$$

in welcher

R für Alkyl, Halogenalkyl oder gegebenenfalls substituiertes Aryl steht, Hal für Chlor oder Brom steht und m und n immer verschieden sind und für

Null oder 2 stehen,

sehr gut zur Regulierung des Pflanzenwachstums geeignet sind.

Überraschenderweise zeigen die erfindungsgemäß zu verwendenden Halogenäthyl-thiosulfonsäureester eine wesentlich bessere Pflanzenwachstumsregulierende Wirksamkeit als die aus dem Stand der Technik bekannten Stoffe (2-Chloräthyl)- trimethylammonium-chlorid und 2- Chloräthylphosphonsäure, welches hochaktive Wirkstoffe gleicher Wirkungsart sind. Die erfindungsgemäße Verwendung der oben definierten Halogenäthyl-thiosulfonsäureester stellt somit eine wertvolle Bereicherung der Technik dar. Die erfindungsgemäß verwendbaren Halogenäthyl-thiosulfonsäureester sind durch Formel (I) definiert. Vorzugsweise steht darin

R für geradkettiges oder verzweigtes Alkyl oder Chloralkyl mit jeweils 1 bis 6, insbesondere mit 1 bis 4 Kohlenstoffatomen oder für Phenyl, welches gegebenenfalls durch Alkyl mit 1 bis 4 Kohlenstoffatomen, insbesondere Methyl und/oder durch Halogen, insbesondere Chlor, substituiert ist,

Hal steht vorzugsweise für Chlor und

m und n, welche immer verschieden sind,

stehen für Null oder 2 .

Als Beispiele für die erfindungsgemäß verwendbaren Verbindungen seien im einzelnen genannt:

2-Chloräthyl-thiosulfonsäure-S-methylester,
2-Chloräthyl-thiosulfonsäure-S-äthylester,
2-Chloräthyl-thiosulfonsäure-S-propylester,
2-Chloräthyl-thiosulfonsäure-S-butylester,
2-Chloräthyl-thiosulfonsäure-S-(2-chloräthyl)-ester,
2-Chloräthyl-thiosulfonsäure-S-phenylester,
2-Chloräthyl-thiosulfonsäure-S-(4-methyl-phenyl)-ester,
2-Chloräthyl-thiosulfonsäure-S-(4-chlorophenyl)-ester,
Methyl-thiosulfonsäure-S-(2-chloräthyl)ester,
Äthyl--thiosulfonsäure-S-(2-chloräthyl)ester,
Propyl-thiosulfonsäure-S-(2-chloräthyl)ester,
Butyl--thiosulfonsäure-S-(2-chloräthyl)ester,
Phenyl-thiosulfonsäure-S-(2-chloräthyl)ester,
(4-Methyl-phenyl)-thiosulfonsäure-S-(2-chloräthyl)ester und
(4-Chloro-phenyl)-thiosulfonsäure-S-(2-chloräthyl)ester.

Einzelne der erfindungsgemäß verwendbaren Verbindungen sind neu; sie können jedoch nach bekannten Verfahren in einfacher Weise hergestellt werden. So erhält man diejenigen Halogenäthyl-thiosulfonsäureester der Formel (I), in denen m für 2 und n für 0 steht, indem man Disulfide der Formel

R - S - S - R (II)

in welcher

R die oben angegebene Bedeutung hat,

mit einer äquimolaren Menge Chlor, gegebenenfalls unter Verwendung eines Verdünnungsmittels, wie Methylenchlorid, bei Temperaturen zwischen $-20^{\circ}C$ und $+ 10^{\circ}C$ umsetzt und dann die dabei entstehenden Sulfensäurechloride der Formel

R - S - Cl (III)

in welcher

R die oben angegebene Bedeutung hat,

mit einer äquimolaren Menge an 2-Halogenäthylsulfinsäuren der Formel

$Hal - CH_2 - CH_2 - SO_2H$ (IV)

in welcher

Hal die oben angegebene Bedeutung hat,

gegebenenfalls in Gegenwart eines Verdünnungsmittels, wie Methylenchlorid, bei Temperaturen zwischen $- 20^{\circ}C$ und $+ 40^{\circ}C$ umsetzt.

In analoger Weise erhält man diejenigen Verbindungen der Formel (I), in denen m für 0 und n für 2 steht, indem man Disulfide der Formel

$Hal - CH_2 - CH_2 - S - S - CH_2 - CH_2 - Hal$ (V)

in welcher

Hal die oben angegebenen Bedeutung hat,

mit einer äquimolaren Menge Chlor gegebenenfalls in Gegenwart eines Verdünnungsmittels, wie Methylenchlorid, bei Temperaturen zwischen -20°C und + 10°C umsetzt und dann die dabei entstehenden Sulfensäurechloride der Formel

$$Hal - CH_2 - CH_2 - S - Cl \qquad (VI)$$

in welcher
Hal die oben angegebene Bedeutung hat,

mit einer äquimolaren Menge an Sulf nsäure der Formel

$$R - SO_2H \qquad (VII)$$

in welcher
R die oben angegebene Bedeutung hat,

gegebenenfalls in Gegenwart eines Verdünnungsmittels, wie Methylenchlorid, bei Temperaturen zwischen - 20°C und + 40°C umsetzt.

Die Aufarbeitung erfolgt jeweils in der Weise, daß man das gesamte Reaktionsgemisch mit Wasser wäscht, die organische Phase abtrennt, trocknet, gegebenenfalls einengt und gegebenenfalls andestilliert.

Die zur Herstellung der erfindungsgemäß verwendbaren Halogenäthyl - thiosulfonsäureester als Ausgangsstoffe benötigten Verbindungen der Formeln (II) bis (VII) sind bekannt oder lassen sich nach im Prinzip bekannten Verfahren herstellen.

Diejenigen Verbindungen der Formel (I), in denen Hal für Chlor oder Brom steht, m für 2 und n für O steht, und R für $CH_2 - CH_2 - Cl$ oder $-CH_2 -CH_2 -Br$ steht, lassen sich auch herstellen, indem man Bis- (2-halogen-äthyl) - disulfide der Formel

$$Hal - CH_2 - CH_2 - S - S - CH_2 - CH_2 - Hal \qquad (VIII)$$

in welcher

Hal die oben angegebene Bedeutung hat,

mit Sulfurylchlorid in Gegenwart von Essigsäure sowie gegebenenfalls in Gegenwart eines weiteren Verdünnungsmittels, wie Methylenchlorid, bei Temperaturen zwischen $-10^{o}C$ und $+20^{o}C$ umsetzt.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff eine oder auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium des Samens oder der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in gewünschter Weise positiv beeinflussen.

Pflanzenwuchsregulierende Stoffe können zum Beispiel zur Hemmung des vegetativen Pflanzenwachstums eingesetzt werden. Eine derartige Wuchshemmung ist unter anderem bei Gräsern von wirtschaftlichem Interesse, denn durch eine Dämpfung des Graswachstums kann z.B. die Häufigkeit der Grasschnitte in Ziergärten, Park- und Sportanlagen oder an Straßenrändern reduziert werden. Von Bedeutung ist auch die Hemmung des Wuchses von krautigen und holzigen Pflanzen an Straßenrändern und in der Nähe von Überlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt.

Eine Hemmung des vegetativen Wachstums erlaubt bei vielen Kulturpflanzen eine dichtere Anpflanzung der Kultur, so daß ein Mehrertrag bezogen auf die Bodenfläche erzielt werden kann.

Ein weiterer Mechanismus der Ertragssteigerung mit Wuchshemmern beruht darauf, daß die Nährstoffe in stärkerem Maße der Blüten- und Fruchtbildung zugute kommen, während das vegetative Wachstum eingeschränkt wird.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, so daß z.B. mehr oder größere Früchte zur Ausbildung kommen.

Ertragssteigerungen können in manchen Fällen auch durch einen Eingriff in den pflanzlichen Stoffwechsel erreicht werden, ohne daß sich Änderungen des vegetativen Wachstums bemerkbar machen. Wachstumsregulatoren können ferner eine Veränderung der Zusammensetzung der Pflanzen bewirken, um so eine bessere Qualität der Ernteprodukte herbeizuführen. So ist es beispielsweise möglich, den Gehalt an Zucker in Zuckerrüben, Zuckerrohr, Ananas sowie Zitrusfrüchten zu erhöhen oder den Proteingehalt in Soja oder Getreide zu steigern.

Unter dem Einfluß von Wachstumsregulatoren kann es zur Ausbildung parthenokarper Früchte kommen. Ferner kann das Geschlecht der Blüten beeinflußt werden.

Mit Wachstumsregulatoren läßt sich auch die Produktion oder der Abfluß von sekundären Pflanzenstoffen positiv beeinflussen. Als Beispiel sei die Stimulierung des Latexflusses bei Gummibäumen genannt.

Während des Wachstums der Pflanze kann durch Einsatz von Wachstumsregulatoren auch die seitliche Verzweigung durch eine chemische Brechung der Apikaldominanz vermehrt werden. Daran besteht z.B. Interesse bei der Stecklingsvermehrung von Pflanzen. Es ist jedoch auch möglich, das Wachstum der Seitentriebe zu hemmen,

z.B. um bei Tabakpflanzen nach der Dekapitierung die Ausbildung von Seitentrieben zu verhindern und damit das Blattwachstum zu fördern.

Unter dem Einfluß von Wachstumsregulatoren kann der Blattbestand von Pflanzen so gesteuert werden, daß ein Entblättern der Pflanzen zu einem gewünschten Zeitpunkt erreicht wird. Eine derartige Entlaubung ist von Interesse, um eine mechanische Beerntung, z.B. bei Wein oder Baumwolle, zu erleichtern oder um die Transpiration zu einem Zeitpunkt herabzusetzen, an dem die Pflanze verpflanzt werden soll.

Durch Einsatz von Wachstumsregulatoren läßt sich der vorzeitige Fruchtfall verhindern. Es ist jedoch auch möglich, den Fruchtfall, - zum Beispiel bei Obst -, im Sinne einer chemischen Ausdünnung bis zu einem bestimmten Ausmaß zu fördern. Wachstumsregulatoren können auch dazu dienen, um bei Kulturpflanzen zum Zeitpunkt der Ernte die zum Ablösen der Früchte erforderliche Kraft zu vermindern, so daß eine mechanische Beerntung der Pflanzen ermöglicht beziehungsweise eine manuelle Beerntung erleichtert wird.

Mit Wachstumsregulatoren läßt sich ferner eine Beschleunigung oder auch eine Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Hilfe von Wachstumsregulatoren auch eine zeitliche Konzentrierung der Reife erzielt werden. Damit werden die Voraussetzungen dafür geschaffen, daß z.B. bei Tabak, Tomaten oder Kaffee, eine vollständige mechanische oder manuelle Beerntung in nur einem Arbeitsgang vorgenommen werden kann.

Durch Anwendung von Wachstumsregulatoren kann auch die Samen- oder Knospenruhe der Pflanzen, also die endogene Jahresrhythmik, beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen.

Mit Wachstumsregulatoren kann auch erreicht werden, daß der Austrieb von Knospen oder die Keimung von Samen verzögert wird, z.B um in frostgefährdeten Gebieten eine Schädigung durch Spätfröste zu vermeiden.

Wachstumsregulatoren können auch eine Halophilie bei Kulturpflanzen erzeugen. Damit werden die Voraussetzungen dafür geschaffen, daß eine Kultivierung von Pflanzen auf salzhaltigen Böden durchgeführt werden kann.

Mit Wachstumsregulatoren kann auch eine Frost- und Trockenresistenz bei Pflanzen induziert werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide; Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Begasen usw. Es ist ferner möglich, die Wirkstoffe nach dem Ultra-Low-Volume-Verfahren auszubringen, Pflanzen oder Pflanzenteile mit der Wirkstoffzubereitung oder dem Wirkstoff selbst zu bestreichen oder die Wirkstoffzubereitung oder den Wirkstoff selbst in den Boden zu injizieren. Es kann auch das Saatgut der Pflanze behandelt werden.

Die Wirkstoffkonzentrationen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach dem klimatischen und vegetativen Gegebenheiten richtet.

Die erfindungsgemäße Verwendung von Halogenäthylthiosulfonsäureestern der Formel (I) wird durch die nachfolgenden Beispiele illustriert.

## Beispiel A

**Stimulation der Äthylenbiosynthese bzw. Äthylenabspaltung**

Lösungsmittel: 30 Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Aus Sojabohnenblättern werden Blattstücke gleicher Größe gestanzt. Diese werden zusammen mit 1 ml der jeweiligen Wirkstoffzubereitung bzw. Kontroll-Lösung in luftdicht verschließbare Gefäße gegeben. Nach einer Stunde werden diese Gefäße verschlossen. Nach weiteren 24 Stunden wird das Äthylen, das sich in den Gefäßen angesammelt hat, mit üblichen Nachweismethoden bestimmt. Die Äthylenentwicklung der mit Wirkstoffzubereitung behandelten Blattstücke wird mit der Äthylenentwicklung der Kontrollen verglichen.

In der nachfolgenden Tabelle bedeuten:

0 Äthylenbildung wie bei der Kontrolle
\+ Äthylenbildung etwas größer als bei der Kontrolle
++ Äthylenbildung größer als bei der Kontrolle
+++ Äthylenbildung viel größer als bei der Kontrolle

Dieser Test ist in besonderem Maße geeignet, die wachstumsregulierenden Eigenschaften der erfindungsgemäßen Verbindungen zu verdeutlichen.

Das Pflanzenhormon Äthylen greift in zahlreiche Prozesse bei der Entwicklung der Pflanzen ein. Eine Erhöhung der Äthylenmenge, wie sie mit den erfindungsgemäßen Substanzen erzielt werden kann, erlaubt es, diese Prozesse zu steuern. Als Beispiele für die möglichen Wirkungen, für die ein besonderes kommerzielles Interesse besteht, seien hier genannt: Fruchtablösung, Reifebeschleunigung von Früchten und Blättern, Blühinduktion, Samenkeimung, Fruchtausdünnung, Stimulation des Latexflusses z. B. bei Hevea, Geschlechtsbeeinflußung und Wuchshemmung z.B. auch um das Lagern von Getreide zu verhindern.

Wirkstoffe, Wirkstoffkonzentrationen und Resultate gehen aus der nachfolgenden Tabelle hervor.

Tabelle A

Stimulation der Äthylenbiosynthese bzw. Äthylenabspaltung

| Wirkstoff | Wirkstoffkonzentration in % | Äthylenbildung |
|---|---|---|
| Kontrolle | — | 0 |
| $Cl-CH_2-CH_2-S(O)_2-S-CH_2-CH_2-Cl$ (8) | 0,001 | + |
| $Cl-CH_2-CH_2-S(O)_2-S-CH_3$ (1) | 0,001 | +++ |
| $Cl-CH_2-CH_2-S(O)_2-S-C_2H_5$ (2) | 0,001 | +++ |
| $Cl-CH_2-CH_2-S-S(O)_2-$ 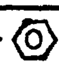 (5) | 0,001 | +++ |
| $Cl-CH_2-CH_2-S-S(O)_2-$ 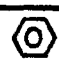 $-Cl$ (6) | 0,001 | +++ |
| $Cl-CH_2-CH_2-S-S(O)_2-$ 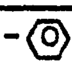 $-CH_3$ (7) | 0,001 | +++ |
| $Cl-CH_2-CH_2-S-S(O)_2-C_2H_5$ (4) | 0,001 | +++ |
| $Cl-CH_2-CH_2-S-S(O)_2-CH_3$ (3) | 0,001 | +++ |

Beispiel B,

Reifebeschleunigung bei Tomaten

Lösungsmittel: 3o Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Im Freiland werden Tomatenpflanzen in üblicher Weise angezogen bis die Hälfte der Früchte gefärbt ist. In diesem Stadium werden die Pflanzen bis zum Abtropfen mit den Wirkstoffzubereitungen besprüht. Nach 2 Wochen wird die Ausfärbung der Früchte im Vergleich zu denen von unbehandelten Kontrollpflanzen bonitiert. Es zeigt sich, daß die erfindungsgemäßen Wirkstoffe der Formeln

$Cl-CH_2-CH_2-S(O)_2-S-CH_2-CH_2-Cl$ (8)

und

$Cl-CH_2-CH_2-S(O)_2-S-C_2H_5$ (2)

eine deutliche Reifebeschleunigung bewirken.

Beispiel C

Wuchsförderung bei Sojabohnen

Lösungsmittel: 30 Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchsförderung % des Zuwachses der Kontrollpflanzen berechnet. Dabei bedeutet 0% ein Wachstum entsprechend dem der Kontrollpflanzen.

Wirkstoffe, Wirkstoffkonzentrationen und Resultate gehen aus der nachfolgenden Tabelle hervor.

Tabelle C

Wuchsförderung bei Sojabohnen

| Wirkstoff | Wirkstoffkonzentration in % | Wuchsförderung in % |
|---|---|---|
| $Cl-CH_2-CH_2-S(O)_2-S-CH_2-CH_2-Cl$ (8) | 0,05 | 20 |
| $Cl-CH_2-CH_2-S(O)_2-S-C_2H_5$ (2) | 0,05 | 15 |
| — (Kontrolle) | — | 0 |

Beispiel D

Wuchsförderung bei Gras (Festuca pratensis)

Lösungsmittel: 30 Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser die gewünschte Konzentration auf.

Gras wird in 7 cm x 7 cm großen Kunststofftöpfen angezogen und bei einer Wuchshöhe von ca. 5 cm mit den Wirkstoffzubereitungen bis zum Abtropfen besprüht. Nach 3 Wochen wird der Zuwachs gemessen und die Wuchsförderung in Prozent des Zuwachses der unbehandelten Kontrollpflanzen berechnet. Dabei bedeutet 0% einen Zuwachs entsprechend dem der Kontrollpflanzen.

Wirkstoffe, Wirkstoffkonzentrationen und Resultate gehen aus der nachfolgenden Tabelle hervor.

Tabelle D

Wuchsförderung bei Gras (Festuca pratensis)

| Wirkstoff | Wirkstoff-konzentration in % | Wuchsförderung in % |
|---|---|---|
| - (Kontrolle) | — | 0 |
| $Cl-CH_2-CH_2-N^{\oplus}(CH_3)_3 \quad Cl^{\ominus}$ (bekannt) | 0,05 | 0 |
| 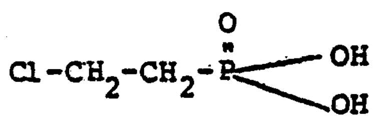 (bekannt) | 0,05 | 0 |
| $Cl-CH_2-CH_2-S(O)_2-S-CH_2-CH_2-Cl$ (8) | 0,05 | 40 |

Beispiel: E

Entblätterung bei Rosen

| | | |
|---|---|---|
| Lösungsmittel: | 30 | Gewichtsteile Dimethylformamid |
| Emulgator: | 1 | Gewichtsteil Polyoxyäthylen-Sorbitan-Monolaurat |

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gleichaltrige Rosenstöcke der Sorte "Queen Elizabeth Rose" werden im Herbst vor dem natürlich einsetzenden Laubfall mit den Wirkstoffzubereitungen bis zum Abtropfen besprüht. Nach 10 Tagen wird der Blattfall im Vergleich zu den Kontrollpflanzen bonitiert.

Der erfindungsgemäße Wirkstoff der Formel

$$Cl-CH_2-CH_2-S(O)_2-S-CH_2-CH_2-Cl \qquad (8)$$

zeigte in diesem Versuch bei 0,2 % Anwendungskonzentration eine sehr gute Wirksamkeit und verursachte bei 0,4 % sogar eine völlige Entlaubung.

Beispiel: F

Fruchtablösung bei Kirschen

Lösungsmittel: 30 Gewichtsteile Dimethylformamid
Emulgator: 1 Gewichtsteil Polyoxyäthylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

An Kirschbäumen der Sorte "Schattenmorelle" werden Zweige mit 30 - 40 Früchten etwa 14 Tage vor der normalen Ernte mit den Wirkstoffzubereitungen bis zum Abtropfen besprüht. Zehn Tage später werden die zur Ablösung der Früchte erforderlichen Kräfte mit einem Zugkraftmesser bestimmt.

Im Vergleich zur Kontrolle zeigte sich an den mit dem erfindungsgemäßen Wirkstoff der Formel

$$Cl-CH_2-CH_2-S(O)_2-S-CH_2-CH_2-Cl \qquad (8)$$

behandelten Zweigen eine deutliche Reduktion der zur Ablösung der Früchte erforderlichen Kräfte.

Herstellungsbeispiele:

Beispiel 1

$$Cl-CH_2CH_2-S(O)_2-S-CH_3$$

In eine Lösung von 18,8 g (0,2 Mol) Dimethyldisulfid in 200 ml Methylenchlorid werden bei einer Innentemperatur von -10°C 14,2 g (0,2 Mol) Chlor eingeleitet. Das Reaktionsgemisch wird noch 30 Minuten bei -10°C gerührt. Die so hergestellte Lösung von Methansulfensäurechlorid wird dann tropfenweise zu einer Lösung von 27 g (0,2 Mol) 2-Chloräthansulfinsäure in 200 ml Methylenchlorid bei einer Innentemperatur von -10°C gegeben. Die komplette Reaktionsmischung wird eine Stunde bei 15 bis 25°C gerührt, dann zweimal mit Wasser gewaschen und mit Natriumsulfat getrocknet. Das Lösungsmittel wird im Vakuum abgezogen und der Rückstand andestilliert. Man erhält 29 g (83 % der Theorie) 2-Chloräthyl-thiosulfonsäure-S-methylester als gelbstichiges Öl.

Brechungsindex: $n_D^{22} = 1,5318$ .

Nach der im Beispiel 1 angegebenen Methode werden auch die in der folgenden Tabelle 1 formelmäßig aufgeführten Verbindungen hergestellt.

Tabelle 1

| Beispiel-Nr. | Wirkstoff | Ausbeute (% der Theorie) | Brechungsindex $n_D^{22}$ |
|---|---|---|---|
| 2 | $Cl-CH_2CH_2-S(O)_2-S-C_2H_5$ | 83 % | $n_D^{22}$=1,5222 |
| 3 | $Cl-CH_2CH_2-S-S(O)_2-CH_3$ | 80 % | $n_D^{22}$=1,5350 |
| 4 | $Cl-(CH_2)_2-S-S(O)_2-C_2H_5$ | 74 % | $n_D^{22}$=1,5282 |
| 5 | $Cl-CH_2CH_2-S-S(O)_2-$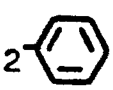 | 71 % | $n_D^{22}$=1,5846 |
| 6 | $Cl-CH_2CH_2-S-S(O)_2-$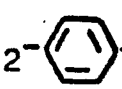$-Cl$ | 82 % | $n_D^{22}$=1,5832 |
| 7 | $Cl-CH_2CH_2-S-S(O)_2-$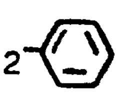$-CH_3$ | 74 % | $n_D^{22}$=1,5818 |

Beispiel 8

$$Cl-CH_2CH_2-S(O)_2-S-CH_2CH_2-Cl$$

Eine Mischung aus 95,5 g (0,52 Mol) Bis-(2-chloräthyl)-disulfid, 30 g Essigsäure und 200 ml Methylenchlorid wird bei einer Innentemperatur zwischen 0 und -5°C mit 135 g (1 Mol) Sulfurylchlorid versetzt. Das Reaktionsgemisch wird eine Stunde bei 0 bis 10°C gerührt, dann bei 10 bis 15°C mit 18 g Wasser und 30 g Essigsäure versetzt und weitere zwei Stunden gerührt.

Es wird dann mehrmals mit Wasser, dann vorsichtig mit Bicarbonatlösung gewaschen, getrocknet und im Vakuum das Lösungsmittel abgezogen.

Man erhält 86 g (81 % der Theorie) 2-Chloräthyl-thiolsulfonsäure-S-(2-chloräthyl)-ester.

Das Produkt erstarrt nach längerem Stehen: Schmelzpunkt 29°C .

Patentansprüche:

1) Mittel zur Regulierung des Pflanzenwachstums, gekennzeichnet durch einen Gehalt an mindestens einem Halogenäthyl-thiosulfonsäureester der Formel

$$Hal-CH_2CH_2-S(O)_m-S(O)_n-R \qquad (I)$$

in welcher

R für Alkyl, Halogenalkyl oder gegebenenfalls substituiertes Aryl steht, Hal für Chlor oder Brom steht und m und n immer verschieden sind und für

Null oder 2 stehen.

2) Verfahren zur Regulierung des Pflanzenwachstums, dadurch gekennzeichnet, daß man Halogenäthyl-thiosulfonsäureester gemäß Anspruch 1 auf die Pflanzen oder ihren Lebensraum einwirken läßt.

3) Verwendung von Halogenäthyl-thiosulfonsäureester gemäß Anspruch 1 zur Regulierung des Pflanzenwachstums.

4) Verfahren zur Herstellung von pflanzenwachstumsregulierenden Mitteln, dadurch gekennzeichnet, daß man Halogenäthyl-thiosulfonsäureester gemäß Anspruch 1 mit Streckmitteln und/oder oberflächenaktiven Mitteln vermischt.